# EUROPEAN PATENT SPECIFICATION

(11) **EP 4 045 022 B1**
(45) Date of publication and mention of the grant of the patent: **09.07.2025**
(21) Application number: 20780933.6
(22) Date of filing: 09.09.2020
(51) Int. Cl.: A61K 31/075, A61K 31/05, A61P 19/02, A61P 25/16, A61P 25/28, A61P 29/00, A61P 35/00, A61P 1/16, A61P 5/06, A61P 3/10, A61P 9/10, A61P 9/12, A61P 19/10, A61P 3/06, A23L 33/125

(54) **COMPOSITION FOR PREVENTING AND TREATING TAUOPATHIES**
ZUSAMMENSETZUNG ZUR VERHINDERUNG UND BEHANDLUNG VON TAUOPATHIEN
COMPOSITION POUR LA PRÉVENTION ET LE TRAITEMENT DES TAUOPATHIES

(30) Priority: 09.09.2019 EP 19382778
(43) Date of publication of application: 24.08.2022
(73) Proprietor: Servicio Andaluz de Salud, 41071 Sevilla (ES); Euronutra, S.L., 29590 Málaga (ES); Universidad de Málaga, 29071 Málaga (ES)
(72) Inventor: RODRIGUEZ DE FONSECA, Fernando, 29010 Málaga (ES); NAVARRO GALERA, Juan Antonio, 29010 Málaga (ES); BAIXERAS LLANO, Elena, 29010 Málaga (ES); DECARA DEL OLMO, Juan Manuel, 29010 Málaga (ES); MEDINA VERA, Dina, 29010 Málaga (ES); LÓPEZ GAMBERO, Antonio Jesús, 29010 Málaga (ES); SUAREZ PEREZ, Juan, 29010 Málaga (ES); SANJUAN MERINO, Carlos, 29590 Málaga (ES); ROSELL DEL VALLE, Cristina, 29010 Málaga (ES); PAVÓN MORÓN, Francisco Javier, 29010 Málaga (ES)
(74) Representative: Hoffmann Eitle
(86) International application number: PCT/EP2020/075180
(87) International publication number: WO 2021/048195

(56) References cited:
- WO-A1-2015/134726
- KR-B1- 101 671 502
- US-A1- 2006 004 096
- US-A1- 2016 151 301
- ORR MIRANDA E ET AL: "A Brief Overview of Tauopathy: Causes, Consequences, and Therapeutic Strategies", TRENDS IN PHARMACOLOGICAL SCIENCES, ELSEVIER, HAYWARTH, GB, vol. 38, no. 7, 25 April 2017 (2017-04-25), pages 637 - 648, XP085076281, ISSN: 0165-6147, DOI: 10.1016/J.TIPS.2017.03.011

## Description

### Field of the invention

This invention relates to the delivery of a composition, preferably a pharmaceutical composition, comprising D-pinitol or any pharmaceutically acceptable salt thereof, for use in the treatment or prevention of disorders, diseases or conditions responsive to the stimulation of the ghrelin receptor in a subject in need thereof.

### Background of the invention

The aging process in humans is associated with physical decline and impairment of metabolic homeostasis (1). While physical decline has a relevant expression on increased fragility and the development of sarcopenia, a generalized loss of muscle mass and muscle cell performance, the dysregulation of the metabolic network leads to age-related increase of obesity, insulin resistance, diabetes and hypertension (2). Ageing impairs the activity of key metabolic signaling pathways and the ensuing metabolic dysregulation results in accelerated ageing. Thus the association of frailty, impaired metabolic homeostasis and hypertension increases vulnerability and limits the quality of life and indeed the life span of the elderly.

A key signaling system impaired in aging is insulin signaling. Age-related impairment in the activity of the insulin signalling pathway results in insulin resistance (1). The ensuing hyperglycemia, as a result of dysregulated glucose clearance, promotes formation of advanced glycation end products (AGEs), which in turn cause tissue damage further exacerbating metabolic dysregulation and accelerating the aging process. A key hepato-muscular loop is profoundly affected by the development of obesity and insulin resistance, where the coupling in between hepatic glucose production and glucose consumption by the muscle is dysregulated as results of the impairment on insulin signaling that leads to overproduction of hepatic glucose, while inhibits its utilization by the muscle, already affected by ageing-associated frailty (1).

Among the different metabolic signalling pathways that control energy homeostasis and muscle vitality, Ghrelin, a peptide hormone produced by specialized cells in the gastrointestinal tract, emerges as a potential target for age-associated metabolic dysregulation and frailty (3-5). Ghrelin is capable of promoting appetite, inhibiting insulin secretion, increasing growth hormone release, enhancing muscle vitality by increasing net muscle mass, and improving cognition (6-9). If we considered that aging produces loss of appetite, excessive insulin secretion an insulin resistance, muscle frailty and cognitive impairment, the physiological effects of Ghrelin is positioned to fight all of these age-associated conditions. Thus, enhancing the release of Ghrelin or the administration of Ghrelin receptor agonists haves been proposed to be used as a method for fighting the above cited age associated conditions (5), including hypertension since decreased Ghrelin levels is positively associated to age-related hypertension (10). ORR MIRANDA E ET AL: "A Brief Overview of Tauopathy: Causes, Consequences, and Therapeutic Strategies", TRENDS IN PHARMACOLOGICAL SCIENCES, ELSEVIER, HAYWARTH, GB, vol. 38, no. 7, 25 April 2017, discloses tauopathies, including chronic traumatic encephalopathy, Pick's disease, corticobasal degeneration, and progressive supranuclear palsy, along with strategies for their treatment using agents such as donepezil, rivastigmine, and memantine, etc.

### Brief description of the invention

The present invention refers to a composition, preferably a pharmaceutical composition or a nutraceutical or a food composition, comprising D-pinitol or any pharmaceutically acceptable salts , esters, tautomers, solvates and hydrates thereof, for use in preventing or slowing the onset of a tauopathy in a subject, wherein the tauopathy is selected from the group consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

In an embodiment, the composition is a pharmaceutical composition which further comprises a pharmaceutically acceptable carrier.

In another embodiment, the composition is a dietary supplement.

In another embodiment, the composition is a dietary supplement.

In another embodiment, the composition is a nutraceutical composition.

In another embodiment, optionally in combination with any of the previous embodiments, the composition is administered orally or via intragastric.

In yet another embodiment, optionally in combination with any of the previous embodiments, the composition is administered to a healthy subject that does not suffer any clinical manifestations of a tauopathy or of mild cognitive impairment.

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

In the context of the present invention, "normal pre-prandial reference levels" are understood as pre-prandial reference levels in a healthy subject. Plasma Levels of Ghrelin are discussed, among others, in European Endocrinology, 2015;11(2):90-5 DOI: 10.17925/EE.2015.11.02.90 In the context of the present invention, "active ghrelin" is understood as the acylated (usually n-octanoylated) form of ghrelin. It is generated as a post-translational esterification of a fatty (n-octanoic or, to a lesser extent, n-decanoic) acid on serine residue at position 3 in the secreted Ghrelin. This acylation is necessary for the activity of ghrelin.

In the context of the present invention, "age related conditions" is understood as any physiological change or pathological disorder that is most often seen with increased senescence. Normal physiological changes associated with aging include loss of muscle mass and increased muscle frailty or mild cognitive impairment. Pathological disorders whose prevalence increased exponentially with age include: atherosclerosis, cardiovascular disease, type 2 diabetes, osteoporosis, hypertension, Alzheimer's disease, arthritis, cataracts and cancer.

In the context of the present invention, "sarcopenia" is defined as the loss of skeletal muscle mass and strength as a result of ageing.

In the context of the present invention, "age related hypertension" is understood as the increase in systolic blood pressure associated with age derived of the age associated increase in total (and renal vascular) resistance as a consequence of the progressive loss of the viscoelastic properties of conduit vessels, increased atherosclerotic arterial disease, and hypertrophy and sclerosis of muscular arteries and arterioles.

### Brief description of the figures

Only Figures relating to the presently claimed D-pinitol form part of the present invention. Figures relating to other inositols are reference figures.

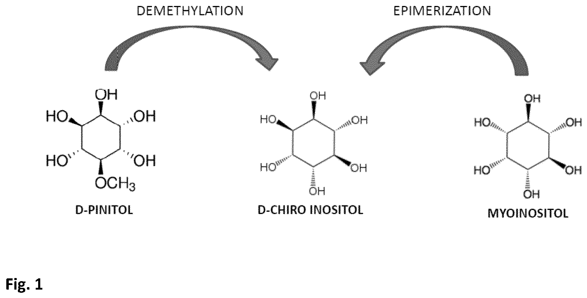

**Figure 1****.** Structure and relationship of D-Pinitol ((1S,2S,4S,5R)-6-methoxycyclohexane-1,2,3,4,5-pentol), D-Chiroinositol (1R,2R,3S,4S,5S,6S)-cyclohexane-1,2,3,4,5,6-hexol and D-myoinositol Myoinositol (1R,2S,3r,4R,5S,6s)-cyclohexane-1,2,3,4,5,6-hexol). Inositols are polialcohols with insulin-mimetic properties. D-Pinitol from natural sources (i.e. carob fruit) can be demethylated in the acid media of the stomach to be converted into D-chiro inositol). In addition, another inositol found in the diet is an isomer of D-chiro inositol that can be converted into D-chiro inositol by means of the enzymatic action of an epimerase.

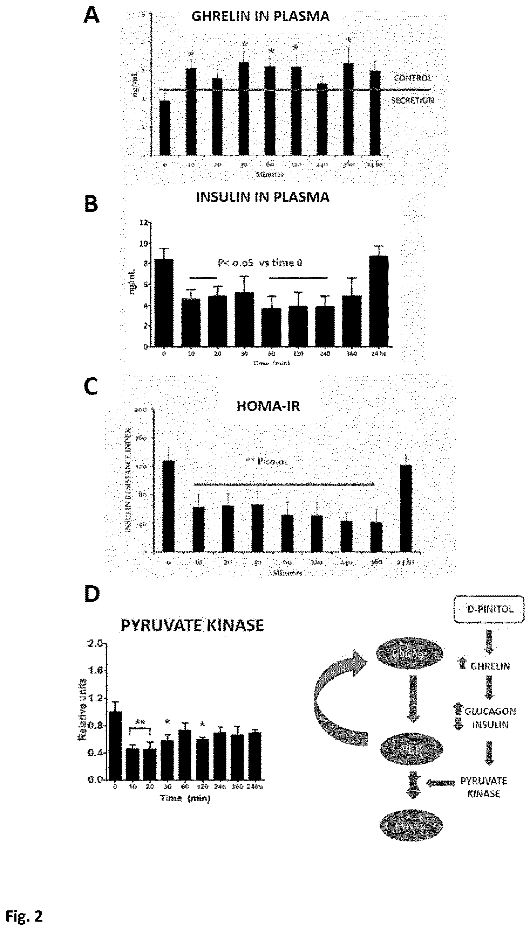

**Figure 2****.** As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

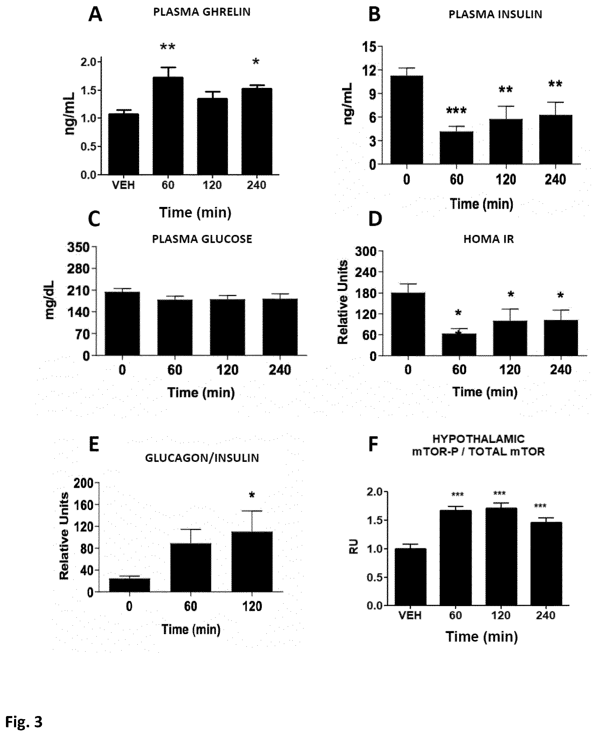

**Figure 3****.** As shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

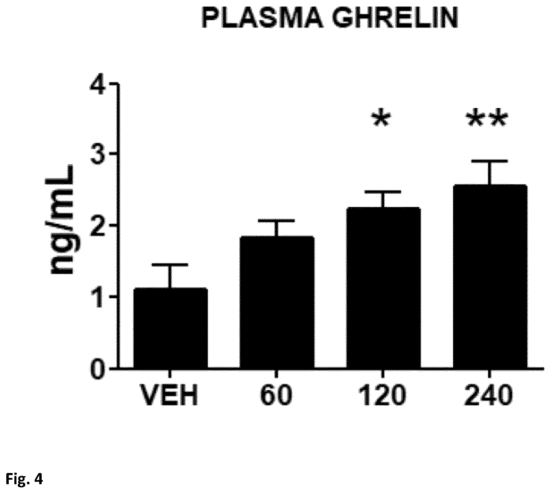

**Figure 4****.** As shown in figure 4, oral administration of D-Chiroinositol (500 mg/kg) enhances Ghrelin secretion as measured through monitorization of circulating plasma Ghrelin concentrations.

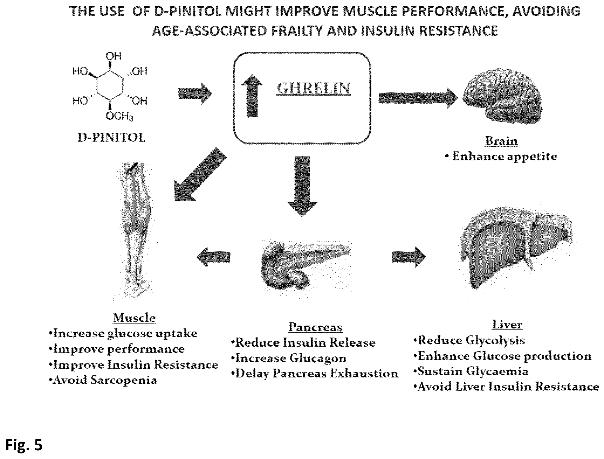

**Figure 5** illustrates a proposed model of action of inositols in metabolic aging. Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

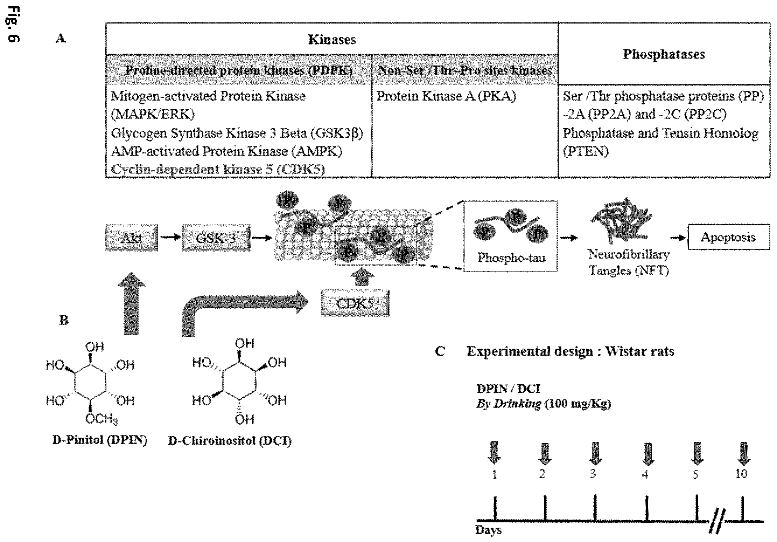

**Figure 6. (A)** Kinases and Phosphatases analysed in this invention by western blotting to evaluate its activity and/or expression. (C) Schematic representation of tau phosphorylation due to the disfunction of the Akt-GSK3 pathway, or the activation of additional kinases such as protein kinase A (PKA) or cyclin-dependent kinase (CDK5). In physiological conditions, Akt is phosphorylated and, therefore activated. In a pathological condition, such as brain insulin resistance, Akt activates the kinase GSK-3β, which in turn, phosphorylates tau. This can occur when other kinases (PKA, CDK5) are hyperactivated When tau protein is hyperphosphorylated, it dissociates from the microtubule and forms insoluble aggregates called neurofibrillary tangles (NFTs) in neurons and glial cells. Both the destabilization of the microtubule and the aggregation of NFTs leads to cell apoptosis. **(B)** Chemical structure of D-Pinitol (DPIN. DPIN is the 3-O-methyl form of D-chiro-inositol (DCI) and is found as a cyclitol, a cyclic polyol. It is a known anti-diabetic agent isolated from the pulp of the carob fruit (*Ceratonia siliqua*)*.* Schematic timeline of the experimental designs: Chronic drinking administration of DPIN and DCI on Wistar rats were performed on 20 male rats for 10 consecutive days. Plasma and brain samples were collected from both experimental groups. Abbreviations: DPIN, D-Pinitol; DCI, D-Chiro-inositol.

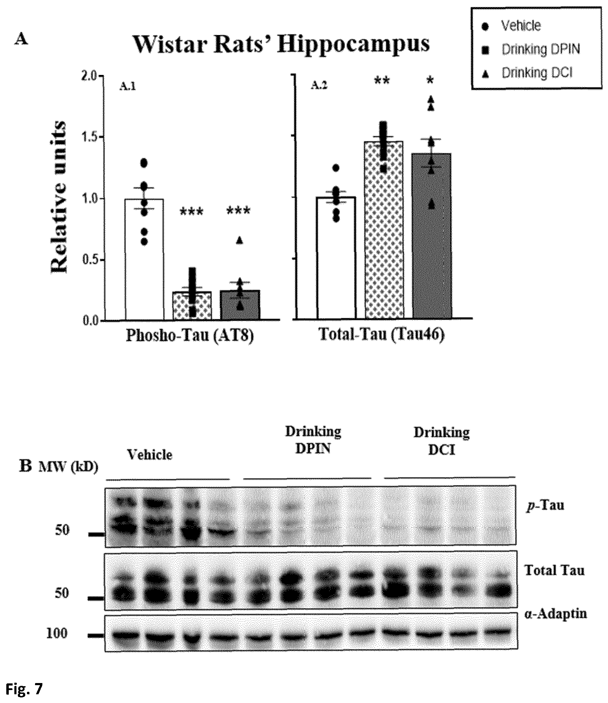

**Figure 7****. Effect of oral administration of DPIN or DCI for 10 days on tau dephosphorization in the hippocampus of Wistar rats.** Bar charts represent the ratio between tau phosphorylation (AT8: Ser202, Thr205) and total tau, and quantity of total tau compared with α-Adaptin on A) Wistar rats, Histograms represent the mean ± S.E.M. (n=8 for Wistar rats). B) Western blot membranes after each antibody incubation (identification target protein right to the band). Molecular weights (MW) are indicated in kilodaltons (kD). The blots shown results from four out of eight independent samples for Wistar rats' groups. The corresponding expression of α-Adaptin is shown as a loading control per lane. One-way ANOVA and Tukey test were performed: (*) P <0.05, (**) P <0.01 and (***) P <0.001 vs Vehicle group.

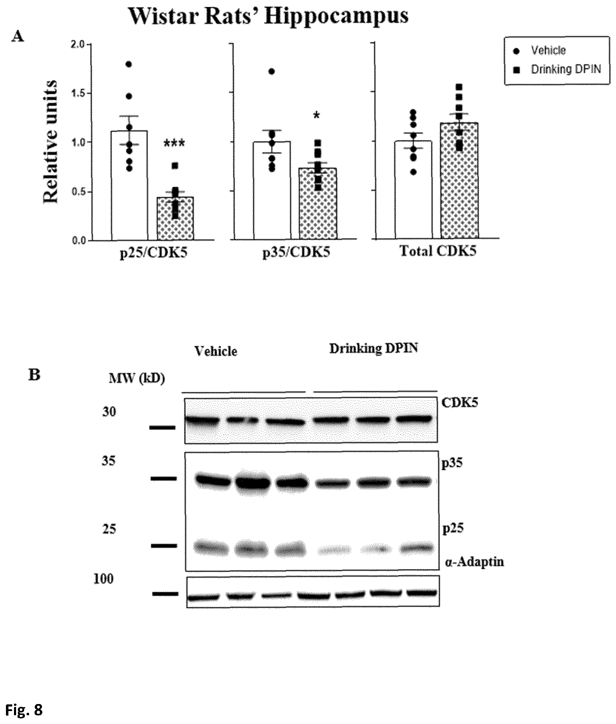

**Figure 8****. Effect of oral administration of DPIN or DCI for 10 days on the tau kinase Cyclin Dependent Kinase 5 (CDK5) activity in the hippocampus of Wistar rats. A)** Bar charts represent the ratio between quantity of the p25, p35 and total CDK5 tau kinase compared with α-Adaptin on Wistar rats. Histograms represent the mean ± S.E.M. (n=8 for Wistar ratsts) **B)** Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

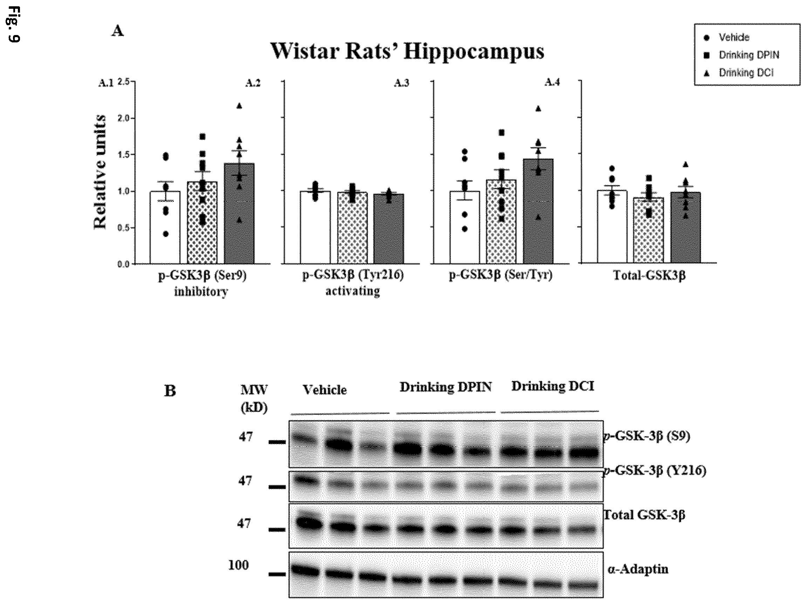

**Figure 9****. Effect of oral administration of DPIN or DCI for 10 days on glycogen synthase kinase-3β (GSK-3 β) phosphorylation in the hippocampus of Wistar rats.** A) Bar charts represent the ratio between GSK-3 β phosphorylation (Ser9/Tyr216) and total GSK3 β, and quantity of total GSK-3 β compared with α-Adaptin on Wistar rats, B) Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

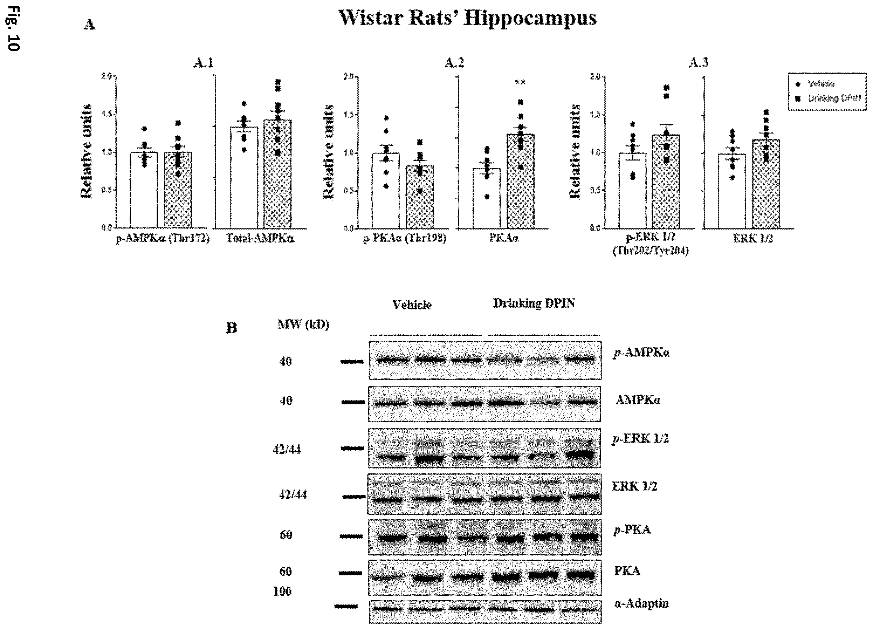

**Figure 10****.** Effect of oral administration of DPIN or DCI for 10 days on other hippocampus's tau kinases: Mitogen-activated Protein Kinase (MAPK), AMP-activated Protein Kinase (AMPK) and Protein Kinase A (PKA) in the hippocampus of Wistar rats. A) Bar charts represent the ratio between quantity of total tau kinases compared with α-Adaptin on Wistar rats. Histograms represent the mean ± S.E.M. (n=8 for Wistar rats). B) Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

### Description of the invention

The present specification generally relates to a method for increasing ghrelin levels as a result of the oral administration of D-pinitol, a natural cyclic polyol derived from plants including the pods of the carob tree (figure 1). Other stereoisomers of inositol, not forming part of the present invention, could be used, such as D-chiro inositol, cis-inositol, epi-inositol, alloinositol, muco-inositol, neo-inositol, L-chiro-inositol, acyllo-inositol, or any of the pharmaceutically acceptable salts, esters, tautomers, solvates and hydrates thereof, or any of the combinations thereof. As shown in the examples included throughout the present specification, the oral administration of D-Pinitol (100 mg/kg (Figure 2) or the oral administration of 500 mg/kg of D-Chiro inositol (Figure 3), enhances ghrelin secretion and reduces circulating insulin, and further activates the phosphorylation of mTOR in the hypothalamus, a metabolic sensor needed to increase appetite (figure 3). The net effect is a reduction of the insulin demand, as reflected by the lowering of the insulin resistance index HOMA (Figures 2 & 3), but without leading to hypo- or hyperglycaemia. This is possible thanks to the direct effect of D-pinitol and D-Chiroinositol on the glucose uptake by muscle cells coupled to the net production of glucose by the liver thanks to the inhibition of the pyruvate kinase that redirects glycolisis towards glucose production in the liver (noeglucogenesis) (figure 2). Administration of D-Chiro inositol also enhances ghrelin secretion (figure 4).

In addition, and as proposed in figure 5, the use of D-Pinitol and D-Chiro inositol to promote Ghrelin secretion harmonizes insulin and glucagon secretion, reducing insulin demand, and redirecting glucose production from the liver to its utilization by the muscle. In addition, because of the known actions of ghrelin, it is expected that D-pinitol or related inositols such as D-chiro inositol or its epimer myo-inositol, increase muscle mass and vitality reducing muscle frailty. Since Ghrelin concentrations decline with age (16), these compounds will help promoting a healthy metabolic aging.

On the other hand, the authors of the present invention have found a role for D-Pinitol in the prevention of cognitive impairment in aging and in tauopathies. In this sense, the insulin saving properties of D-pinitol and its ability for enhancing Ghrelin secretion, has a procognitive effect that can be used as a preventive strategy for cognitive impairment associated to neurodegeneration. Insulin resistance in the brain is associated to cognitive impairment, in special by modifying hippocampal function. On the other side, Ghrelin is a unique hormone capable of enhancing cognition through its ability of crossing the blood-brain barrier and interaction with the Growth hormone secretagogue receptor. Thus, the novel action described in the present invention might account for the use of D-pinitol administration in aging or in neurodegenerative disorders, especially Alzheimer's Disease to reduce cognitive impairment in early stages of the disease, when symptoms allows diagnosis.

However, recent studies have highlighted the need on focusing in a molecular target that is the best associated factor to the onset of mild cognitive impairment and the development of major dementias: the tau protein. Abnormal phosphorylation or acetylation of tau generates tau protein deposition, causing the appearance of neurofibrillary tangles (NFT) as an hystopathological biomarker of a group of diseases collectively known as tauopathies. The formation of NFT is more strongly correlated with cognitive decline than the distribution of senile plaque, which is formed by polymorphous beta-amyloid (Ax) protein deposits, a pathological hallmark of one of the main dementias, Alzheimer's disease. Tau deposition is thus the crucial element for the cognitive impairment observed in Alzheimer's disease (where β-amyloid deposition is insufficient to generate dementia as well as in the progression towards mild cognitive impairment and the dementia observed in chronic traumatic encephalopathy).

**Thus, tauopathy has been considered as an essential hallmark in neurodegeneration and normal brain aging, and its prevention before the appearance of clinical symptoms an uncovered medical need.** Taking the above into consideration, we examined the effects of the administration of D-Pinitol on the phosphorylation state of Tau, a complex process regulated by multiple proteins (Figure 6) and surprisingly found that the oral administration of D-Pinitol reduced markedly the phosphorylation of Tau (Figure 7), through a mechanism dependent of the reduction on the activity of the cyclin-dependent kinase 5 (Figure 8), one of its major phosphorylation enzymes. D-Pinitol actions were specific since they did not affect other tauregulatory proteins (Figure 9), providing a unique pharmacological profile for this natural inositol.

Based on the above findings, in the present invention we further propose that the administration of D-Pinitol shall
a) prevent or retard the onset of mild cognitive impairment and its ulterior progression to dementia if a condition is promoting tau hyperphosphorylation and there is an intention of preventing or retarding its clinical appearance. These conditions include Pick disease, progressive supranuclear palsy, corticobasal degeneration, argyrophilic grain disease, globular glial tauopathies, primary age-related tauopathy, which includes neurofibrillary tangle dementia, chronic traumatic encephalopathy (CTE), and aging-related tau astrogliopathy.
b) Treat diagnosed tauopathies, including Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

Therefore, the present disclosure generally relates to the inositol D-pinitol (from hereinafter compound of the present invention) for increasing ghrelin levels. The compound of the present invention is described by the structural formula identified in figure 1 or by any pharmaceutically acceptable salt derived therefrom. Moreover, the present invention further relates to the compound of the present invention to reduce the phosphorylation of Tau and thus preventing or slowing the progression of the clinical manifestations of a tauopathy in a subject, preferably in a subject before the appearance of such clinical manifestations, more preferably in a human subject, wherein the tauopathy is selected from the group consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

In the present invention, the term "asymptomatic subject" is understood as a subject that does not show the clinical manifestation of the disease, in particular of a tauopathy.

In the present invention, the term "onset of the clinical manifestation of a tauopathy" is understood as the appearance of cognitive, neuropsychological and/or neurological symptoms and signs, including objective diagnostic procedures (i.e. magnetic resonance imaging, positron emission tomography and cerebrospinal fluid biomarkers) that indicates the diagnostics of a tauopathy.

In the present invention, the term "the onset of the clinical manifestations of mild cognitive impairment" (MCI) is understood as the stage between the expected cognitive decline of normal aging and the more serious decline of dementia. It's characterized by problems with memory, language, thinking or judgment.

In the present invention the term "prevention" means to avoid occurrence of the disease or pathological condition in an individual, particularly when the individual has predisposition for the pathological condition but has not yet been diagnosed. In the present invention, the disease or pathological condition is preferably a "tauopathy".

In the present invention, the term "tauopathy" belongs to a class of neurodegenerative diseases involving the aggregation of tau protein into neurofibrillary or gliofibrillary tangles (NFTs) in the human brain. Tangles are formed by hyperphosphorylation of the microtubule protein known as tau, causing the protein to dissociate from microtubules and form insoluble aggregates. (These aggregations are also called paired helical filaments.) Examples of tauopathies are selected from the list consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

As already indicated, the compound of the present invention is useful to markedly reduce the phosphorylation of Tau and thus preventing or slowing the onset or progression of the clinical manifestations of a tauopathy in a subject, preferably in a subject before or after the appearance of such clinical manifestations. Therefore, a further aspect of the present invention provides a method for preventing or slowing the onset or progression of the clinical manifestations of a tauopathy in a subject. More preferably, the present invention provides a method for preventing or slowing the onset of the clinical manifestations of a tauopathy in a subject, that is, a method for preventing or slowing the appearance of such clinical manifestations in said subject, preferably in a healthy subject.

Yet another aspect of the present invention relates to the use of D-pinitol or any pharmaceutically acceptable salt thereof for the manufacture of a medicament useful for preventing or slowing the progression of the clinical manifestations of a tauopathy in a subject, that is, for preventing or slowing the appearance of such clinical manifestations in said subject, preferably in a healthy subject.

In addition to what has been described above, the present invention also encompasses the possibility that a composition of the present invention is in the form of a dietary supplement or nutritional composition that comprises D-pinitol or any salt thereof. In this sense, in the event that the composition of the invention is formulated as a nutritional composition, said nutritional composition may be a food or be incorporated into a food or food product intended for both human and animal consumption. Thus, in a particular embodiment, the nutritional composition is selected from between a food (which may be a food for specific nutritional purposes or medicinal food) and a nutritional supplement.

In the present invention, the term "nutritional composition" refers to that food, which regardless of providing nutrients to the subject who consumes it, beneficially affects one or more functions of the body, so as to provide better health and wellness.

The term "supplement", synonymous with any of the terms "dietary supplement", "nutritional supplement", "food supplement", or "alimentary supplement" or "alimentary complement" refers to products or preparations whose purpose is to supplement the normal diet consisting of sources of concentrated nutrients or other substances with a nutritional or physiological effect. In the present invention, the "substance" which has a nutritional or physiological effect on the individual when the alimentary complement is ingested is D-pinitol or any salt thereof, which is part of any of the compositions of the present invention. The food supplement may be in single or combined form and be marketed in dosage form, i.e. in capsules, pills, tablets and other similar forms, sachets of powder, ampoules of liquids and drop dispensing bottles and other similar forms of liquids and powders designed to be taken in a single amount. The food supplement maybe any plant-derived supplement containing D-Pinitol, including carob fruitderived syrups.

Preferably, said nutritional compositions or dietary supplements are intended or use for preventing or slowing the progression of the clinical manifestations of a tauopathy in a subject, preferably in a subject before or prior to the appearance of such clinical manifestations, more preferably in a healthy subject.

There is a wide range of nutrients and other elements that may be present in alimentary complements including, among others, vitamins, minerals, amino acids, essential fatty acids, fiber, enzymes, plants and plant extracts. Since their role is to complement the supply of nutrients in a diet, they should not be used as a substitute for a balanced diet and intake should not exceed the daily dose expressly recommended by the doctor or nutritionist.

Examples of foods that may comprise the compositions of the invention include, but not limited to, feed, dairy products, vegetable products, meat products, snacks, chocolates, drinks, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverage may be, but is not limited to, non-fermented milk. In a particular embodiment, the food product or food is selected from the group consisting of fruit or vegetable juices, ice cream, infant formula, milk, yogurt, cheese, fermented milk, powdered milk, lyophilized or air-dried products (suitable for reconstitution with a liquid vehicle), cereals, baked goods, milk-based products, meat products and beverages.

D-pinitol or any pharmaceutically acceptable salt thereof or ester compounds can be provided in a kit. Such a kit typically contains an active compound of D-pinitol in dosage forms for administration. A dosage form contains a sufficient amount of active compound such that a beneficial effect can be obtained when administered to a subject during regular intervals, such as 1, 2, 3, 4, 5 or 6 times a day, during the course of 1 or more days. Preferably, a kit contains instructions indicating the use of the dosage form and the amount of dosage form to be taken over a specified time period.

The term "subject" means a mammal. One embodiment of the term "mammal" is a "human," said human being either male or female. As such, the term "mammal" includes companion animals such as cats and dogs. The term "mammal in need thereof refers to a mammal who is in need of treatment or prophylaxis as determined by a researcher, veterinarian, medical doctor or other clinician.

The term "composition", as in pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier. The term "composition" is also intended to encompass nutraceutical or food compositions. A nutraceutical is a food or food product that provides health and medical benefits, including the prevention and treatment of human ailments.

It will be understood that the compounds of the present invention include hydrates, solvates, polymorphs, crystalline, hydrated crystalline and amorphous forms of the compounds of the present invention, and pharmaceutically acceptable salts thereof. The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, lithium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl- morpholine, N-ethylpiperidine, glucamine, glucosamine, histidine, hydrabamine, isopropylamine, lysine, methylglucamine, moϕholine, piperazine, piperidine, polyamine resins, procaine, purines, theobromine, triethylamine, trimethylamine, tripropylamine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic acid, trifluoroacetic acid, and the like. Particularly preferred are citric, fumaric, hydrobromic, hydrochloric, maleic, phosphoric, sulfuric, and tartaric acids.

Preferably, and as already indicated throughout the present specification, it will be understood that, as used herein, references to compounds D-pinitol are meant to also include the pharmaceutically acceptable salts, such as the hydrochloride salts. The compound of the present invention is useful in the treatment, control or prevention of diseases, disorders or conditions characterized by an impaired ghrelin secretion resulting in active ghrelin blood circulation levels lower than normal pre-prandial active ghrelin blood circulation reference levels.

The terms "administration of and or "administering" a compound should be understood to mean providing a compound of the invention to a subject in need of treatment. The administration of the compounds of the present invention in order to practice the present methods of therapy is carried out by administering a therapeutically effective amount of the compound to a subject in need of such treatment or prophylaxis. The need for a prophylactic administration according to the methods of the present invention is determined via the use of well-known risk factors.

The term "therapeutically effective amount" as used herein means the amount of the active compound that will elicit the biological or medical response in a tissue, system, subject, mammal, or human that is being sought by the researcher, veterinarian, medical doctor or other clinician, which includes alleviation of the symptoms of the disorder being treated. The novel methods of treatment of this invention are for disorders known to those skilled in the art. The term "prophylactically effective amount" as used herein means the amount of the active compound that will elicit the biological or medical response in a tissue, system, subject, mammal, or human that is being sought by the researcher, veterinarian, medical doctor or other clinician, to prevent the onset of the disorder in subjects as risk for obesity or the disorder. The therapeutically or prophylactically effective amount, or dosage, of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

Administration and Dose Ranges. Any suitable route of administration may be employed for providing a subject or mammal, especially a human with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the invention are administered orally. The effective dosage of active ingredient employed may vary depending on the particular compound employed, the mode of administration, the condition being treated, and the severity of the condition being treated. Such dosage may be ascertained readily by a person skilled in the art.

The magnitude of prophylactic or therapeutic dosage of the compounds of the present invention will, of course, vary depending on the particular compound employed, the mode of administration, the condition being treated, and the severity of the condition being treated. It will also vary according to the age, weight and response of the individual patient. Such dosage may be ascertained readily by a person skilled in the art.

As already stated, compounds of the invention may be used in combination with other drugs that are used in the treatment/prevention/suppression or amelioration of the diseases or conditions for which the compound of the invention is useful. Such other drugs may be administered, by a route and in an amount commonly used therefor, contemporaneously or sequentially with a compound of the invention. When a compound of the invention is used contemporaneously with one or more other drugs, a pharmaceutical composition containing such other drugs in addition to the compound of the invention is preferred.

The compositions of the present invention include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy. In practical use, the compounds of the invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the typical oral dosage unit form, in which case solid pharmaceutical carriers are typically employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray. The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of the invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

Finally, compounds of the invention may also be administered as nutraceutical or food compositions such as beverages.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

The following examples serve to illustrate the present invention but do not limit the same.

### Examples

Only Examples relating to the presently claimed D-pinitol form part of the present invention. Examples relating to other inositols, i.e. D-chiro-inositol, are reference examples.

### Example 1.

### METHODOLOGIY OF EXAMPLE 1

### Animals and ethics statement.

Experimental procedures with animals were carried out in strict accordance with the recommendations in the European Communities directive 2010/63/EU and Spanish legislation (Real Decreto 53/2013, BOE 34/11370-11421, 2013) regulating the care and use of laboratory animals. The protocol was approved by the Ethics Committee for Animal Experiments of the University of Malaga. All studies involving animals were reported in accordance with the ARRIVE guidelines for reporting experiments involving animals (Kilkenny, C., Browne, W. J., Cuthill, I. C., Emerson, M. & Altman, D. G. Improving Bioscience Research Reporting: The ARRIVE Guidelines for Reporting Animal Research. PLoS Biol 8, e1000412, https://doi.org/10.1371/journal.pbio.1000412 (2010). All efforts were made to minimize animal suffering and to reduce the number of animals used. The experiments were performed on 4-to-5-week-old male Wistar rats (Crl:WI; Charles River Laboratories, Barcelona, Spain). The animals were under a standard 12 h light-dark cycle in a room with temperature and humidity control. All the rats were provided with water and commercially available rat standard pellet diet (STD) (3.02 kcal/g with 30 kcal% protein, 55 kcal% carbohydrates and 15 kcal% fat) ad libitum.

### Drug preparation and dose administered.

D-Pinitol (3-0-methyl-D-chiro-inositol) was generously provided by EURONUTRA SL (https://www.euronutra.com/, Málaga, Spain), in the form of crystalline fine powder (lot: PPN-M0201). For acute treatments, D-Pinitol was dissolved in water to be administered by gavage (orally) at different concentrations (100 mg/Kg and 500 mg/Kg), drug was administered at a volume of 1 ml/kg.

### Treatment guidelines.

After 18 hours of fasting, D-Pinitol dissolved in water was administered acutely by gavage (100 mg/kg in a first study, and 500 mg/kg in a subsequent study). After administration, the animals were sacrificed in groups at different times (being time 0 the administration of D-Pinitol). For the first study (100 mg/kg), groups of animals were sacrificed at times: 10, 20, 30, 60, 120, 240 and 360 minutes after administration; for the second study (500 mg/kg), animals were sacrificed at times: 60, 120 and 240 minutes after administration. As a control group, a group was administered only with water (by gavage).

### Sample collection.

Animals were anesthetized with sodium pentobarbital (50 mg/Kg, i.p.), blood, brain and liver samples were collected. Blood was centrifuged (2100 g for 8 min, 4 °C) and the plasma kept for further analysis. Liver and brain samples were flash-frozen in liquid N2, then stored at -80 °C until further analysis.

### Measurement of metabolites in plasma.

The plasma levels of insulin and ghrelin were determined with an enzyme-linked immunosorbent assay (ELISA) method using commercial kits: EMD Millipore Corporation (Billerica, MA, USA) Cat. #EZRMI-13K and Cat. #EZRGRT-91K, respectively. Glucagon levels in plasma was determined with a Glucagon Enzyme Immunoassay (EIA) Kit: Cat. #RAB0202 Sigma-Aldrich (Saint Louis, MO, USA). All serum samples were assayed in duplicate within one assay, and results were expressed in terms of the particular standard hormone.

### RNA isolation and cDNA synthesis.

Total RNA was extracted from tissue portions of liver (100-300 mg) using the Trizol^{®} method according to the manufacturer's instructions (GIBCO BRL Life Technologies). To ensure the purity of the mRNA sequences, RNA samples were isolated with a RNeasy Minelute Cleanup Kit (Qiagen), which included digestion with DNase I column (RNase-free DNase set, Qiagen), according to the manufacturers' instructions. Total RNA was quantified using a spectrophotometer (Nanodrop 1000 Spectrophotometer, Thermo Scientific) to ensure A260/280 ratios of 1.8 to 2.0. Reverse transcription was carried out from 1 µg of RNA using the Transcriptor Reverse Transcriptase kit and random hexamer primers (Transcriptor RT, Roche Diagnostic GmbH). Negative controls included reverse transcription reactions that omitted the reverse transcriptase.

### Real-time Quantitative Polymerase Chain Reaction (qPCR) and Gene Expression Analysis.

Real-time qPCR was performed following the criteria of the MIQE guidelines (Bustin, S. A. et al. The MIQE guidelines: minimum information for publication of quantitative real-time PCR experiments. Clin Chem. 55, 611-622 (2009)). Polymerase chain reactions were carried out on the CFX96 Touch^{™} Real-Time PCR detection system (Bio-Rad, Hercules, CA) for each cDNA template, and amplified in 20 µl reaction volume containing 9 µl of cDNA (diluted 1/100) and 11 µl of master mix containing the primer (TaqMan, Life Technologies). The target rat gen was Pyruvate Kinase Liver/RBC (Pklr) (Supplementary Table). The primer was obtained based on TaqMan^{®} Gene Expression Assays and the FAM^{™} dye label format (Life Technologies). Each reaction was run in duplicate. Cycling parameters were 50 °C for 2 min to deactivate single- and double stranded DNA containing dUTPs, 95 °C for 10 min to activate Taq DNA polymerase followed by 40 cycles at 95 °C for 15 sec for cDNA melting, and 60 °C for 1 minute to allow for annealing and the extension of the primers, during which fluorescence was acquired. Raw fluorescence data were submitted to the online Miner tool (http://www.miner.ewindup.info/) for calculation of Cq and efficiency values for each experimental set (Zhao, S. & Fernald, R. D. Comprehensive Algorithm for Quantitative Real-Time Polymerase Chain Reaction. J Comput Biol. 12, 1047-1064 (2005)). Cq values were converted into relative expression values taking into account amplification efficiencies, inter-run variations, and normalization factors by means of Biogazelle's qbasePLUS software (Biogazelle, Zwijnaarde, Belgium) using at least two reference rat genes: beta Actin (Actb) and Glyceraldehyde-3-phosphate Dehydrogenase (Gapdh) (Supplementary Table). For all reference and target gene studies, two independent biologic samples of each experimental condition were evaluated in technical duplicates. Repeatability between replicates was accepted when the ΔCq value was ≤0.7. Finally, Calibrated Normalized Relative Quantity (CNRQ) values were exported from the qbasePLUS software and investigated statistically.

**Table 1. Primer references for TaqMan^{®} Gene Expression Assays (Applied Biosystems).**

| **Gene description** | **Assay ID** | **N° accession GenBank** | **Amplicon Length** |
|---|---|---|---|
| **Target genes** | | | |
| *Pklr* | Rn01455286_m1 | NC_005101.4 | 58 |
| **Reference genes** | | | |
| Gapdh | Rn01775763_g1 | NC_005103.4 | 174 |
| Actb | Rn00667869_m1 | NM_031144.2 | 91 |

### Protein extraction and Western blot analysis.

Brain extract. Frozen brain samples (17 mg per sample) were homogenized in 1mL of cold RIPA lysis buffer (50 mM Tris-HCl pH 7.4, 150 mM NaCl, 0.5% NaDOC, 1 mM EDTA, 1% Triton, 0.1% SDS, 1 mM Na3VO4, 1 mM NaF) supplemented with a protease cocktail (Roche). The suspension was incubated for 2 hours at 4°C, followed by centrifugation at 12,000 rpm for 15 minutes at 4 °C. The supernatant was transferred to a new clean centrifuge tube, and Bradford colorimetric method was used to determine the concentration of the total protein. The protein extracts were diluted 1:1 in loading buffer (DTT 2X) and heated for 5 minutes at 99 °C before being subjected to electrophoresis.

Western Blot Analysis. The expression of protein including mTOR (289kDa) and Adaptin (100kDa) were analysed by western blot. The tissue protein (10-15µg) was subjected to electrophoresis on 4-12% Criterion XT Precast Bis-Tris gels (Bio-Rad, Hercules, CA, EE.UU.) for 30 minutes at 80V and 2 h at 150V. Proteins were transferred onto a 0.2 µm nitrocellulose membrane (Bio-Rad, Hercules, CA, EE.UU.) for 1h at 80V by wet transfer equipment. The membrane was washed twice for 5 min in TBST (10 mM Tris-HCl, 150 mM NaCl, 0.1% Tween 20, pH 7.6) and blocked with 5% BSA-TBST for one hour at room temperature on a shaker platform. Subsequently, the membrane was incubated with respective primary antibodies overnight at 4°C diluted in 2% BSA-TBST. Antibodies against p-mTOR (Ser2448) and m-TOR were purchased from Cell Signaling Technology (Danvers, Massachusetts, United States); α-Adaptin from Abcam (Cambridge, United Kingdom). The following day, the membrane was washed three times for 5 min with TBST. An appropriate HRP conjugated rabbit/mouse secondary antibody (Promega, Madison, WI, EE.UU.) was diluted 1:10000 in 2% BSA-TST and incubated with the membrane for 1h shaking at room temperature. Finally, the membrane was washed as above and exposed to chemiluminescent reagent (Santa Cruz, Biotechnology Inc., CA, EE.UU.) for 5 min. Respective membrane bound protein was then visualized by Chemiluminescence (ChemiDoc Imaging System, Bio-Rad). Bands were quantified by densitometric analysis using ImageJ software (Rasband, WS, ImageJ, US National Institutes of Health, Bethesda, MD, USA).

All data are expressed as mean ± SEM. Statistical analysis were conducted in GraphPad Prism, version 8 (GraphPad Software, Inc., La Jolla, CA). One-way analysis of variance (ANOVA) was assessed, followed by a Turkey's Multiple Comparisons Test when appropriate. P values less than 0.05 were considered significant.

### Results of example 1

### 1.1. Results of the oral administration of Pinitol (100 mg/kg) dissolved in sterile water to adult male Wistar Rats

As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

Furthermore, as shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

### 1.2. Results of the oral administration of D-Chiroinositol (500 mg/kg) dissolved in sterile water to adult male Wistar Rats

As shown in figure 4, oral administration of D-Chiroinositol (500 mg/kg) enhances Ghrelin secretion as measured through monitorization of circulating plasma Ghrelin concentrations.

### 1.3. Proposed model of action of inositols in metabolic aging

Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

### Example 2

### METHODOLOGY

### Animals and ethics statement

Animal experimental procedures were carried out in accordance with the European Communities directive 2010/63/EU and Spanish legislation (Real Decreto 53/2013). The protocol was approved by the Research and approved by Ethics Committee for Animal Experiments of the University of Malaga, Spain. In accordance with the ARRIVE guidelines, all efforts were made to minimize animal suffering and to reduce the number of animals used per experimental group. Chronic drinking experiments on Wistar rats were performed on 20 male rats. All adult rats, ageing 2 months (≈300 g of body weight), were provided by Charles River Laboratories (Barcelona, Spain). The animals were housed individually under a standard 12 h light-dark cycle in a room with temperature and humidity control. Water and rat chow pellets were provided ad libitum throughout the course of the present study.

### Preparation and administration of inositols

Caromax^{®}-D-Pinitol (3-O-methyl-d-chiro-inositol, DPIN, 98% purity) and Caromax^{®}-D-Chiroinositol (cis-1,2,3-trans-3,5,6-cyclohexanehexol, DCI) were provided by Euronutra SL (Málaga, Spain). They were dissolved in water to orally daily administration by drinking and/or gavage at dose of 100 mg/Kg of body weight (BW) in a 1 ml/kg of BW volume to Zucker and Wistar rats for 4 weeks (28 days) and 10 days, respectively (Figure 1). During the drinking treatments, the concentration of inositols in the water were updated considering the daily increase of BW of the rats and the possible loss of water by evaporation.

### Protein extraction and Western blot analysis.

- **Brain extract.** Frozen brain samples (17 mg per sample) were homogenized in 1mL of cold RIPA lysis buffer (50 mM Tris-HCl pH 7.4, 150 mM NaCl, 0.5% NaDOC, 1 mM EDTA, 1% Triton, 0.1% SDS, 1 mM Na3VO4, 1 mM NaF) supplemented with a protease cocktail (Hoffmann-Roche). The suspension was incubated for 2 hours at 4°C, followed by centrifugation at 12,000 rpm for 15 minutes at 4°C. The supernatant was transferred to a new clean centrifuge tube, and Bradford colorimetric method was used to determine the concentration of the total protein. The protein extracts were diluted 1:1 in loading buffer (DTT 2X) and heated for 5 minutes at 99 °C before being subjected to electrophoresis.
- **Western Blot Analysis.** The tissue protein (10-15 µg) was subjected to electrophoresis on 4-12% Criterion XT Precast Bis-Tris gels (Bio-Rad, USA) for 30 minutes at 80V and 2 h at 150V. Proteins were transferred onto a 0.2 µm nitrocellulose membrane (Bio-Rad, USA) for 1h at 80V by wet transfer equipment. The membrane was washed twice for 5 min in TBST (10 mM Tris-HCl, 150 mM NaCl, 0.1% Tween 20, pH 7.6) and blocked with 2 % Bovine Serum Albumin-Tris Buffered Saline Tween 20 (BSA-TBST) for one hour at room temperature on a shaker platform. Subsequently, the membrane was incubated with respective primary antibodies overnight at 4°C diluted in 2% BSA-TBST (Table 2 for additional information). The following day, the membrane was washed three times for 5 min with TBST. An appropriate HRP conjugated rabbit/mouse secondary antibody (Promega) was diluted 1:10000 in 2% BSA-TST and incubated with the membrane for 1h shaking at room temperature. Finally, the membrane was washed as above and exposed to chemiluminescent reagent (Santa Cruz, Biotechnology Inc.) for 5 min. Stripping/reproving steps were used when necessary. Respective membrane bound protein was then visualized by Chemiluminescence (ChemiDoc Imaging System, Bio-Rad). Bands were quantified by densitometric analysis using ImageJ software (Rasband, WS, ImageJ, US National Institutes of Health, Bethesda, MD, USA). Normalization was performed using a reference protein of the same membrane, the α-Adaptin. The results were expressed as the protein / α-Adaptin ratio or phosphorylated / total protein ratio and normalized to the control group (Y axis represents 'fold mean of the control values').

### Data analysis and statistics

All data are expressed as mean ± SEM. Statistical analysis was undertaken for studies where each group size was of, at least, n = 5. Statistical analysis was conducted in GraphPad Prism, version 8 (GraphPad Software, Inc., San Diego, CA, USA). One- and two-way analysis of variance (ANOVA) was assessed, followed by Turkey's *post hoc* multiple comparisons test. The post hoc tests were conducted only if F in ANOVA achieved a P value less than 0.05 and there was no statistically significant variance inhomogeneity. The analysis of two single groups was performed using Student's unpaired t-test. The results were considered statistically significant at P<0.05.

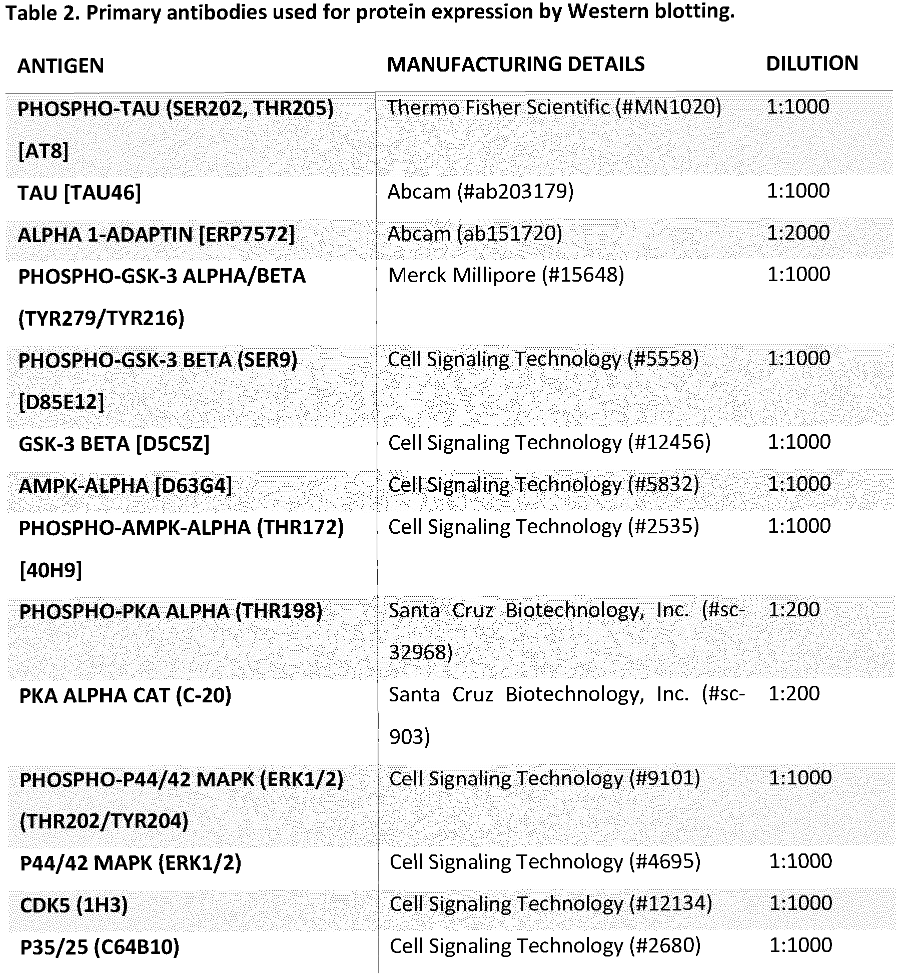

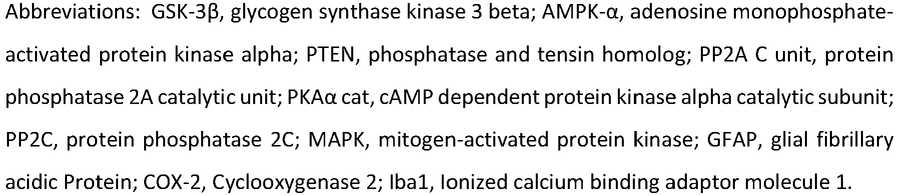

### RESULTS

### 2.1. Effect of D-Pinitol or D-Chiro inositol administrated chronically for 10 days on phosphorylation state of tau protein in the hippocampus of Wistar rats.

Wistar rats were treated orally by drinking DPIN and DCI for 10 days (Figure 6). Here, we analysed the expression and phosphorylation of tau protein, a hallmark of neurodegenerative disorders, such as Alzheimer's disease. For that, we used two specific antibodies: phospho-tau (AT8) antibody which recognizes phosphorylated tau protein in serine 202 and threonine 205. Total Tau (Tau46) antibody recognizes both phosphorylated and non-phosphorylated isoforms. The chronical administration of DPIN and DCI had a statistically significant effect on tau protein dephosphorylation. Quantity of phospho-tau after both compounds has dropped to more than half compared with vehicle group (one-way ANOVA: F_{(2,23)} =49.97, P value <0.0001; Tukey test: P value <0.0001, Figure 7A.1).

We next quantified how much of total tau protein was in the same samples, in order to evaluate whether that drop of phospho-tau was due to less quantity of total tau or not. Significant statistical differences in the relative protein levels of total tau were found after DPIN and DCI treatment (one-way ANOVA: F_{(2,23)} =11.72, P value =0.0003; Tukey test: P value <0.01, Figure 7A.2) reflecting a rise compared with the vehicle group. Therefore, the chronic administration of DPIN and DCI in Wistar rats for 10 days, not only produced a significant reduction of phospho-tau, but also an increase of the total protein amount.

### 2.2. Cyclin Dependent Kinase 5 (CDK5) inhibition after DPIN administration as a putative explanation of hippocampal tau dephosphorylation.

Cyclin-dependent kinase 5 (cdk5) is believed to be involved in the phosphorylation of tau protein. We studied the expression of the protein levels of CDK5, its activator subunit p35 and the truncated form of p35, p25. In Wistar rats, the oral administration, by drinking, of DPIN significantly decreased the amount of the p25 (unpaired t-test: t= 4.869, df=14; P value =0.0002; Figure 8A) as well as the p35 subunit (unpaired t-test: t= 2.245, df=15; P value =0.0402; Figure 8A). The total amount of CDK5 does not vary (unpaired t-test: t= 1.623, df=14; P value =0.1268; Figure 8A). These results indicate a clear inactivation of the CDK5 kinase in Wistar rats after DPIN administration.

### 2.3. Glycogen synthase kinase-3 beta (GSK-3β) activity is not affected in the hippocampus of Wistar after DPIN treatment.

Due to the interaction between the kinase activity of GSK-3β and phosphorylation of the tau protein, we analysed the expression and phosphorylation of this kinase protein. It is well recognized that serine-9 (S9) phosphorylation in GSK-3β causes N-terminal tail to act as pseudo substrate, hindering binding of the actual substrates, thus inhibiting GSK-3. This inhibition of activity is translated into a decrease of tau phosphorylation, as being GSK-3β one of the main tau kinases. In this example, we aimed to study the role of this tau kinase (GSK-3β) in Wistar rats.

Oral administration of either, DPIN or DC,I had no effect either on serine-9 phosphorylation (one-way ANOVA: F_{(2,22)} =1.809, P value =0.1874; Figure 9A.1) or tyrosine-216 (one-way ANOVA: F_{(2,22)} =1.088, P value =0.3543; Figure 9A.2). This means that there is neither enhance nor inhibition of the kinase activity (one-way ANOVA: F_{(2,22)} =2.473, P value =0.1074; Figure 9A.3). In terms of relative protein levels of total GSK-3β, no significant statistical differences were found after the three compounds administration (one-way ANOVA: F_{(2,22)} =0.5308, P value =0.5955; Figure 9A.4). Therefore, in terms of action on GSK-3β in the hippocampus of Wistar rats, neither drinking DPIN or DCI for 10 days does any effect.

### 2.4. AMP-activated Protein Kinase (AMPK), Protein Kinase A (PKA) and Mitogen-activated Protein Kinase (MAPK / ERK1/2) are not related to hippocampal tau dephosphorylation.

Kinase activation and total expression of Mitogen-activated Protein Kinase (MAPK / ERK1/2), AMP-activated Protein Kinase (AMPK) and Protein Kinase A (PKA) were evaluated to analyse whether those kinases were involved in the above tau results. As shown in Figure 10, Wistar rats presents a significant increase of the total amount of PKA with drinking DPIN administration (unpaired t-test: t= 3.795, df=14; P value =0.0020; Figure 10A.2). No other changes are observed among the other kinases.

### References

1.- Bettedi L, Foukas LC. Growth factor, energy and nutrient sensing signaling pathways in metabolic ageing. Biogerontology. 2017 Dec;18(6):913-929.
2.- Soriguer F, et al. Prevalence of diabetes mellitus and impaired glucose regulation in Spain: the Di@bet.es Study. Diabetologia. 2012 Jan;55(1):88-93.
3.- Kojima M, Hosoda H, Date Y, Nakazato M, Matsuo H, Kangawa K (December 1999). "Ghrelin is a growth-hormone-releasing acylated peptide from stomach". Nature. 402 (6762): 656-60
4.- Seim I, Amorim L, Walpole C, Carter S, Chopin LK, Herington AC (January 2010). "Ghrelin gene-related peptides: multifunctional endocrine / autocrine modulators in health and disease". Clinical and Experimental Pharmacology & Physiology. 37 (1): 125-31
5.- Strasser F. Clinical application of ghrelin. Curr Pharm Des. 2012;18(31):4800-12.
6.- Castañeda TR, Tong J, Datta R, Culler M, Tschöp MH (January 2010). "Ghrelin in the regulation of body weight and metabolism". Frontiers in Neuroendocrinology. 31 (1): 44-60.
7.- Inui A, Asakawa A, Bowers CY, Mantovani G, Laviano A, Meguid MM, Fujimiya M (March 2004). "Ghrelin, appetite, and gastric motility: the emerging role of the stomach as an endocrine organ". The FASEB Journal. 18 (3): 439-56.
8.- Alamri BN, Shin K, Chappe V, Anini Y. The role of ghrelin in the regulation of glucose homeostasis. Horm Mol Biol Clin Investig. 2016 Apr 1;26(1):3-11.
9.- Pena-Bello L, Pertega-Diaz S, Outeiriño-Blanco E, Garcia-Buela J, Tovar S, Sangiao-Alvarellos S, Dieguez C, Cordido F. Effect of oral glucose administration on rebound growth hormone release in normal and obese women: the role of adiposity, insulin sensitivity and ghrelin. PLoS One. 2015 Mar 17;10(3):e0121087.
10.- Leinonen T, Antero Kesäniemi Y, Hedberg P, Ukkola O. Serum ghrelin and prediction of metabolic parameters in over 20-year follow-up. Peptides. 2016 Feb;76:51-6.
11.- Martins L, Fernández-Mallo D, Novelle MG, Vázquez MJ, Tena-Sempere M,Nogueiras R, López M, Diéguez C. Hypothalamic mTOR signaling mediates the orexigenic action of ghrelin. PLoS One. 2012;7(10):e46923.
12.- Yada T, Damdindorj B, Rita RS, Kurashina T, Ando A, Taguchi M, Koizumi M, Sone H, Nakata M, Kakei M, Dezaki K. Ghrelin signalling in β-cells regulates insulin secretion and blood glucose. Diabetes Obes Metab. 2014 Sep;16 Suppl 1:111-7.
13.- Kurashina T, Dezaki K, Yoshida M, Sukma Rita R, Ito K, Taguchi M, Miura R, Tominaga M, Ishibashi S, Kakei M, Yada T. The β-cell GHSR and downstream cAMP/TRPM2 signaling account for insulinostatic and glycemic effects of ghrelin. Sci Rep. 2015 Sep 15;5:14041.
14.- Dang NT, Mukai R, Yoshida K, Ashida H. D-pinitol and myo-inositol stimulate translocation of glucose transporter 4 in skeletal muscle of C57BL/6 mice. Biosci Biotechnol Biochem. 2010;74(5):1062-7.
15.- Yap A, Nishiumi S, Yoshida K, Ashida H. Rat L6 myotubes as an in vitro model system to study GLUT4-dependent glucose uptake stimulated by inositol derivatives. Cytotechnology. 2007 Dec;55(2-3):103-8.
16.- Nass R, Farhy LS, Liu J, Pezzoli SS, Johnson ML, Gaylinn BD, Thorner MO. Age-dependent decline in acyl-ghrelin concentrations and reduced association of acyl-ghrelin and growth hormone in healthy older adults. J Clin Endocrinol Metab. 2014 Feb;99(2):602-8.

## Claims

1. A composition, a pharmaceutical composition or a nutraceutical or food composition or dietary supplement, comprising D-pinitol or any salt thereof, for use in preventing or slowing the onset of a tauopathy in a subject, wherein the tauopathy is selected from the group consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

2. The composition for use according to claim 1, wherein the composition is a pharmaceutical composition which further comprises a pharmaceutically acceptable carrier.

3. The composition for use according to claim 1, wherein the composition is a dietary supplement.

4. The composition for use according to claim 1, wherein the composition is a nutraceutical composition.

5. The composition for use according to any of claims 1 to 4, wherein said composition is administered orally or via intragastric.

6. The composition for use according to any of claims 1 to 5, wherein the composition is administered to a healthy subject that does not suffer any clinical manifestations of a tauopathy.

## Patentansprüche

1. Zusammensetzung, pharmazeutische Zusammensetzung oder nutrazeutische oder Nahrungsmittelzusammensetzung oder Nahrungsergänzungsmittel, umfassend D-Pinitol oder irgendein Salz desselben, für deren Verwendung bei der Verhinderung oder Verzögerung des Ausbruchs einer Tauopathie in einem Individuum, wobei die Tauopathie aus der Gruppe bestehend aus Pick-Krankheit, progressiver supranukleärer Blickparese, kortikobasaler Degeneration, Silberkornkrankheit, globulären glialen Tauopathien, primärer altersbedingter Tauopathie, welche neurofibrillärer Tangle-Demenz, chronischtraumatischer Enzephalopathie (CTE) und altersbedingter Tau-Astrogliopathie umfasst, ausgewählt wird.

2. Zusammensetzung für deren Verwendung nach Anspruch 1, wobei die Zusammensetzung eine pharmazeutische Zusammensetzung ist, welche zusätzlich einen pharmazeutisch akzeptablen Träger umfasst.

3. Zusammensetzung für deren Verwendung nach Anspruch 1, wobei die Zusammensetzung ein Nahrungsergänzungsmittel ist.

4. Zusammensetzung für deren Verwendung nach Anspruch 1, wobei die Zusammensetzung eine nutrazeutische Zusammensetzung ist.

5. Zusammensetzung für deren Verwendung nach einem der Ansprüche 1 bis 4, wobei die genannte Zusammensetzung oral oder intragastral verabreicht wird.

6. Zusammensetzung für deren Verwendung nach einem der Ansprüche 1 bis 5, wobei die Zusammensetzung einem gesunden Individuum verabreicht wird, welches nicht unter irgendeiner klinischen Erscheinungsform einer Tauopathie leidet.

## Revendications

1. Composition, composition pharmaceutique ou composition nutraceutique ou alimentaire ou complément alimentaire, comprenant du D-pinnitol ou tout sel de celui-ci, pour son utilisation dans la prévention ou le ralentissement de l'apparition d'une tauopathie chez un sujet, dans laquelle la tauopathie est choisie parmi le groupe constitué par la maladie de Pick, la paralysie supranucléaire progressive, la dégénérescence corticobasale, la maladie des grains argyrophiles, les tauopathies gliales globulaires, la tauopathie primaire liée à l'âge comportant la démence à enchevêtrement neurofibrillaire, l'encéphalopathie traumatique chronique (ETC), et l'astrogliopathie tau liée au vieillissement.

2. Composition pour son utilisation selon la revendication 1, dans laquelle la composition est une composition pharmaceutique qui comprend en outre un véhicule pharmaceutiquement acceptable.

3. Composition pour son utilisation selon la revendication 1, dans laquelle la composition est un complément alimentaire.

4. Composition pour son utilisation selon la revendication 1, dans laquelle la composition est une composition nutraceutique.

5. Composition pour son utilisation selon l'une quelconque des revendications 1 à 4, dans laquelle ladite composition est administrée par voie orale ou intragastrique.

6. Composition pour son utilisation selon l'une quelconque des revendications 1 à 5, dans laquelle la composition est administrée à un sujet sain que ne souffre pas d'aucune manifestation clinique d'une tauopathie.
